# FASCICULE DE BREVET EUROPEEN

(11) **EP 0 441 070 B1**
(45) Date de publication et mention de la délivrance du brevet: **15.02.1995**
(21) Numéro de dépôt: 90400305.0
(22) Date de dépôt: 05.02.1990
(51) Int. Cl.: A61H 39/08, A61M 5/178

(54) **Dispositif comportant une aiguille percée et un réservoir**
Vorrichtung mit einer hohlen Nadel und einem Reservoir
Device with a hollow needle and a reservoir

(43) Date de publication de la demande: 14.08.1991
(73) Titulaire: Cohen, André, Dr., F-30000 Nimes (FR)
(72) Inventeur: Cohen, André, Dr., F-30000 Nimes (FR)
(74) Mandataire: Manresa Val, Manuel

(56) Documents cités:
- DE-A- 1 771 016
- FR-A- 2 635 003

## Description

L'invention se rapporte aux dispositifs destinés à apporter localement un produit soit médicamenteux, soit quelconque, et comportant un réservoir associé à une aiguille percée d'un canalicule.

Les dispositifs connus du genre en question sont :
1) du type seringue qui comporte une aiguille percée, de grand diamètre (supérieur à 0,3 mm), associée à un réservoir à piston, également de grandes dimensions.
   Utilisée en injection médicamenteuse (substance homéopatique, vitamines, oligo-éléments...) ou en chirurgie esthétique (collagène...), elle provoque des douleurs et des inconvénients pratiques (tiraillement de la peau, doses difficiles à miniaturiser...) liés aux dimensions de l'aiguille et au poids du réservoir;
2) du type décrit dans le brevet US-A-4508119 (K. TUKAMOTO) qui concerne soit une aiguille du type utilisée en acupuncture, en alliage spécial, préalablement magnétisée et soumise ensuite à l'action d'un champ électrostatique, soit une aiguille à injection ordinaire, également pré-magnétisée et soumise à un champ électro-statique, servant à véhiculer un produit médicamenteux préalablement magnétisé dans une boîte magnétique.

Le document DE-A-1771016 décrit on ensemble formé par un réservoir relié à une canule d'injection qui, à cause de ses dimensions, ne peut pas être utilisé pour l'acupuncture
Dans les deux cas les aiguilles et les réservoirs sont de grandes dimensions.

C'est pour remèdier à ces divers inconvénients, que le déposant, docteur en médecine et acupuncteur, a mis au point un ensemble "aiguille percée-réservoir" répondant parfaitement aux besoins de l'acupuncture, de la micro-injection médicamenteuse et de la micro-chirurgie esthétique avec des extensions possibles en micro-mécanique.
A partir d'une aiguille du type utilisé en acupuncture (diamètre inférieur à 0,3 mm), percée, associée à un réservoir de diamètre extérieur inférieur à 15 mm et de courte longueur, il est possible :
a) d'utiliser les aiguilles en acupuncture puis en micro-injection thérapeutique au cours de la même séance;
b) d'utiliser le dispositif en micro-injection thérapeutique ou en micro-chirurgie esthétique.
Ledit dispositif est en outre d'un maniement simple, peu encombrant et quasi-non-traumatisant sur le plan des tissus comme sur celui de la tolérance pour le patient.

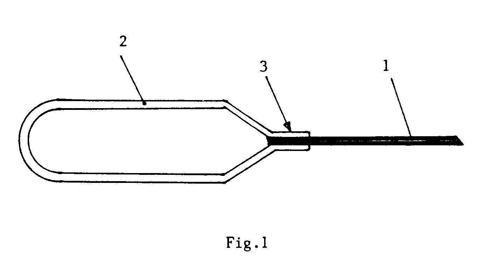

Les caractéristiques et les avantages de l'invention selon la revendication 1 vont apparaître plus clairement à la lecture de la description détaillée qui suit d'un mode de réalisation préféré de l'invention donné à titre d'exemple seulement et représenté au dessin annexé qui est une vue en coupe du dispositif selon l'invention.

Le dispositif, selon l'invention, comporte :
a) une aiguille (1), du type utilisé en acupuncture, de diamètre extérieur compris entre 0,15 et 0,3 mm et de longueur comprise entre 3 et 30 mm (non limitatif), mettant en oeuvre divers métaux dont notamment du fer, du molybdéne, de l'acier écroui, de l'inox, de l'acier japonnais, percée d'un canalicule apte à laisser passer un produit liquide ou visqueux;
2) un réservoir (2), sans piston, contenant ledit produit, réalisé en matériau déformable (plastique, métallique...) par simple pression digitale, d'un diamètre extérieur inférieur à 15 mm dont notamment 5 mm environ, de longueur comprise par exemple entre 20 et 30 mm, recouvert éventuellement, partiellement ou totalement, par un matériau (fil en spirale par exemple) venant renforcer sa structure ou faciliter sa préhension.
La liaison aiguille-réservoir peut se faire par simple emmanchement, le maintien de l'aiguille et du réservoir pouvant se faire par la pression résultant du fait que le diamètre extérieur de l'aiguille peut être supérieur au diamètre intérieur de l'embout (3) du réservoir dans lequel son extrémité se loge.

Bien entendu, l'invention n'est pas limitée au mode de réalisation décrit et représenté pour lequel on pourra d'autres variantes et l'étendre à d'autres applications sans pour cela sortir du cadre de l'invention.
En particulier :
- le moyen de fixation de l'aiguille et du réservoir pourra être des types généralement utilisés pour les seringues;
- le réservoir pourra avoir n'importe quelle forme;
- il pourra s'interchanger en cours de séance.

## Revendications

1. Dispositif, destiné aux soins par acupuncture et par injection médicamenteuse, caractérisé en ce qu'il comporte un ensemble constitué par:
un réservoir, en matériau déformable par simple pression digitale, qui possède un diamètre extérieur inférieur à 15 mm, pourvu d'une ouverture situé à l'une de ses extrémités permettant leur démontage en cours de séance d'acupuncture pour y introduire une substance traitante par l'intermédiaire de ladite ouverture;
une aiguille d'acupuncture, qui possède un diamètre extérieur inférieur à 0,3 mm et qui est percée d'un canalicule apte à laisser passer ladite substance traitante, contenue dans ledit réservoir, auquel elle est rendue solidaire par emmanchement axial forcé dans ladite ouverture, dans le but de compléter les effets énergétiques de ladite aiguille d'acupuncture.

2. Dispositif, selon la revendication 1, caractérisé en ce que ledit réservoir est recouvert, partiellement ou totalement d'un matériau venant renforcer sa structure.

3. Dispositif, selon la revendication 1, caractérisé en ce que ledit réservoir est recouvert partiellement ou totalement d'un matériau venant faciliter sa préhension.

## Claims

1. Device, for acupuncture and injecting treatment, characterised in that it comprises an assembly constituted by:
- a tank, in a material deformable under finger pressure, having an outside diameter inferior to 15 mm, provided with an opening at one of its ends enabling its removal along an acupuncture session in order to introduce a treating substance inside said tank through said opening;
- an acupuncture needle, having an outside diameter inferior to 0,3 mm provided with a lengthwise conduit allowing a free passage therethrough of said treating substance, contained inside said tank, said acupuncture needle being axially fitted into said opening, in order to provide a perfection of the energetic effects of said acupuncture needle.

2. Device, according to claim 1, characterised in that said tank is partially o completely covered by a material which reinforces its structure.

3. Device, according to claim 1, characterised in that said tank is partially o completely covered by a material making its grasp easy.

## Patentansprüche

1. Vorrichtung für Akupunkturbehandlung und zum Einspritzen von Medikamenten, dadurch gekennzeichnet daß sie eine Baugruppe bildet, bestehend aus:
- ein Behälter, aus einem Material, das auf Fingerdruck nachgibt, der einen Außendurchmesser von Weniger als 15 mm aufzeigt, mit einer an einem Ende befindlichen Öffnung, welche die Entfernung des Behälters bei einer Akupunktursitzung erlaubt, um die für die Behandlung notwendige Substanz durch die besagte Öffnung einzuführen;
ein Akupunkturnadel, die einen Außendurchmesser von weniger als 0,3 mm aufweist, die einen längenmäßig verlaufenden Kanal aufweist, um die für die Behandlung notwendige Substanz, die sich im Behälter befindet, durchzulassen, diese Akupunkturnadel wird in die besagte Öffnung des Behälters eingesteckt, mit dem Ziel, die energetischen Auswirkungen der besagten Akupunkturnadel zu ergänzen.

2. Vorrichtung nach Anspruch 1, dadurch gekennzeichnet, daß der besagte Behälter voll oder teilweise mit einem verstärkenden Material verkleidet ist.

3. Vorrichtung nach Anspruch 1, dadurch gekennzeichnet, daß der besagte Behälter eine Komplette oder teilweise Verkleidung aus einem leicht greifbaren Material aufweist.
